# EUROPEAN PATENT SPECIFICATION

(11) **EP 2 645 893 B1**
(45) Date of publication and mention of the grant of the patent: **25.02.2015**
(21) Application number: 11723614.1
(22) Date of filing: 29.03.2011
(51) Int. Cl.: A41D 13/12, A61B 5/024, A61B 5/026

(54) **WHS ITEM OF CLOTHING FOR DETECTION OF VITAL PARAMETERS OF A BABY**
WHS-KLEIDUNGSSTÜCK ZUR ERFASSUNG VON VITALPARAMETERN EINES SÄUGLINGS
ARTICLE VESTIMENTAIRE D'UN SYSTÈME MÉDICAL AMBULATOIRE POUR LA DÉTECTION DES PARAMÈTRES VITAUX D'UN BÉBÉ

(30) Priority: 03.12.2010 IT MI20102245
(43) Date of publication of application: 09.10.2013
(73) Proprietor: Comftech S.r.L., 20900 Monza (MB) (IT); Politecnico di Milano, 20133 Milano (IT)
(72) Inventor: ANDREONI, Giuseppe, I-20835 Muggiò (MB) (IT); MOLTANI, Lara, Alessia, Laura, I-20900 Monza (MB) (IT); ZANINI, Rinaldo, I-23900 Lecco (LC) (IT); BELLU', Roberto, Carlo, I-20038 Seregno (MI) (IT)
(74) Representative: Ciceri, Fabio
(86) International application number: PCT/IB2011/000671
(87) International publication number: WO 2012/073076

(56) References cited:
- EP-A1- 2 191 737
- GB-A- 2 396 043
- GB-A- 2 447 237
- US-A1- 2004 210 165
- US-B1- 6 228 106

## Description

### FIELD OF INVENTION

The present invention relates to an item of clothing for detection of vital parameters of a baby, preferably but without limitation, an item of clothing for premature babies.

### RELATED PRIOR ART

In recent years, advances in treatment of diseases in newborns and premature babies (i.e. born before the thirty-seventh week of pregnancy) have considerably increased life expectancy with an increase of survival rates to 33% for babies born at 23 weeks' gestation and have reduced the negative impact of preterm on neurological, motor and behavioral development of premature babies.

Continuous monitoring of vital parameters of high-risk babies is the main resource through which physicians and experts of the field can retrieve important data on their health and assess their growth day by day.

Integration of detected data allows optimized targeting of diagnostic and therapeutic measures on these little patients.

Premature babies are particularly exposed to external stimuli, which are often excessive and painful for them, and have a fragile clinical picture, due to immature development of their organs, that must complete their development outside the womb, in a hostile environment, with no mechanical, acoustic or visual filtering.

To date, treatment of premature babies requires monitoring of four basic parameters, that fully describe the baby's health state:
- Cardiac monitoring;
- Respiratory monitoring;
- Transcutaneous O2-CO2 monitoring;
- Pulse oximetry.

Full neonatal care requires redundant error handling in health monitoring, whereby the baby is surrounded by systems (e.g. temperature sensors) that can detect the same parameters at several points, or multiple alarm systems are used (e.g. the ventilator alarm and the pulse oximeter alarm).

The devices with which it is connected often cause discomfort to the baby and fill the space in which he/she lies. For this reason, the need is strongly felt for analysis and validation of non-intrusive monitoring devices.

The above drawback has been obviated by the introduction of Wearable Health Systems (WHS); particularly, WHS may be defined as systems integrated on wearable platforms (i.e. garments and body-attached devices), that may provide continuous monitoring solutions through non-invasive measurement of bioelectrical, biochemical and physical parameters.

Therefore, WHS are electronic devices that can be integrated in external garments as parts or accessories thereof. They allow acquisition of vital parameters, such as ECG recordings, and hence heart rate, respiration rate, oxygen saturation and temperature.

The integration of this information allows physicians and users to set up a general picture on the health conditions of the individual under examination.

The demand for WHS has been rapidly increasing, not only in the medical field, but also in ergonomics, occupational medicine, wellness and sport.

The specifications that apply to WHS are:
- Simple use;
- Low weight;
- Small size;
- Elimination or reduction of the wires connected to the patient.

US patent 4,729,377 results from a MIT-DARPA-Georgia Tech research and discloses a garment containing multiple conductive elements in contact with the skin and connected through conductive paths to a single electronic device. The garment could be used both for monitoring signals from detection sites and for stimulation.

The document WO 98/41279 introduces inductive plethysmography, a technique that provides time and volume parameters for inspiration and expiration.

The document WO 0102052 discloses the way of making woven electrodes and introduces the concept of elasticity as a condition to improve the contact between the garment and the body and the electrode and the skin.

The document WO 2004/08968 discloses a structure designed for measuring, processing and transmitting data indicative of the health state of the patient, thanks to the multiplicity of conductive paths, the plurality of electrodes and conductive elements.

Document US 6 228 106 B1 discloses a baby pyjama of electronic textile type comprising a section that can be stretched in at least one direction, said stretchable section being designed to remain in a fixed position relative to said at least one arm and a sensor being coupled in said stretchable section so that it can be properly positioned relative to said arm of the baby to provide an electrical output signal indicative of the vital parameter being detected.

Therefore, WHS allow remote or real-time monitoring and provide screening of the cardiopulmonary activity, temperature, thoracic and abdominal movements.

Two kinds of fibers are used to form integrated sensors: a silver-coated polyamide fiber and a fiber formed of stainless steel and continuous metal of copper, steel, Ag, Ti filaments.

The former provides considerable advantages over the latter, in terms of conductivity and adaptability, but also has drawbacks such as oxidation, low wash-resistance and possible allergic reactions.

In elastic conductive threads, mechanical deformation is shown to affect the flux of charge carriers in the structure.

The sensors have characteristics depending on the structure and geometry of each thread and hence the fabric: the latter is a mechanical stress transducer for a given range of deformations.

The threads that are used for the electronic fabric have various electric properties and may be used and woven in various manners, to provide different structures depending on the desired functions.

While WHS have advantageous characteristics, they are still affected by drawbacks, such as the placement of the sensor relative to the body of the baby whose physiological parameters have to be monitored.

Particularly, in prior art WHS devices, the sensor/s are placed at regions of the body of the baby that should be readily accessed by medical staff in case of emergency. This would require the medical staff to stop WHS monitoring, for instance by removing the WHS.

The WHS usually consists of a dress or a T-shirt or similar garment, and the sensor/s are placed at the chest, to allow, for instance, electrocardiogram recording.

Nevertheless, in an emergency, i.e. if the medical staff has to take urgent action, for instance auscultation of the heart with a stethoscope, they first have to remove the dress or lift up the T-shirt and then lay the stethoscope on the chest. This will stop monitoring of the parameters by the WHS, as contact is discontinued between the skin and the sensor, and further involves an undesired time loss, with obvious imaginable consequences.

### SUMMARY OF THE INVENTION

In the light of the above described prior art, the object of the present invention is to provide an item of clothing for detecting vital parameters of a premature baby, which facilitates action by the health care staff without stopping the detection of the vital parameters of the baby.

According to the present invention, this object is fulfilled by an item of clothing for detecting vital parameters of a baby as claimed in the annexed claim 1.

With the present invention, an item of clothing or WHS may be provided, such as a so-called "baby pyjama" of electronic textile type, preferably designed to be worn by premature babies.

The present invention provides an item of clothing having electrodes located on the sleeves only, which can detect various parameters, including an electrocardiographic derivative, with improved accuracy as compared with the prior art.

Furthermore, the present item of clothing results from accurate wearability design considerations, and further has non-intrusive (and hence non-invasive) and comfort features, with all arrangements and care for the characteristics of the class of individuals for whom it is designed.

In one embodiment, the item of clothing provides optimized configuration of electrodes, to minimize and eliminate artifacts. For this purpose, the textile sensors are fixed to the sleeves of the pyjama, therefore in contact with the baby's arms.

Proper fixation of the sensing fabric to the baby's arms, as well as optimization of the position of the terminals are peculiar features of the item of clothing of the present invention.

In another peculiar aspect of the present embodiment, a conductive yarn was selected for contact with the baby's skin which, in addition to being anallergic, antibacterial, and adapted to be washed, ironed and sterilized according to standard procedures as used in the art, is also softer, to prevent abrasions of the baby's skin and injuries, especially during long-time monitoring.

### BRIEF DESCRIPTION OF THE DRAWINGS

The characteristics and advantages of the invention will appear more clearly from the following description of a practical embodiment, illustrated by way of a non-limiting example in the annexed drawings, in which:
- Figure 1 shows a first embodiment of the item of clothing for detecting vital parameters of a baby, according to the present invention;
- Figures 2A to 2C show three embodiments of a detail of the item of clothing of Figure 1 respectively;

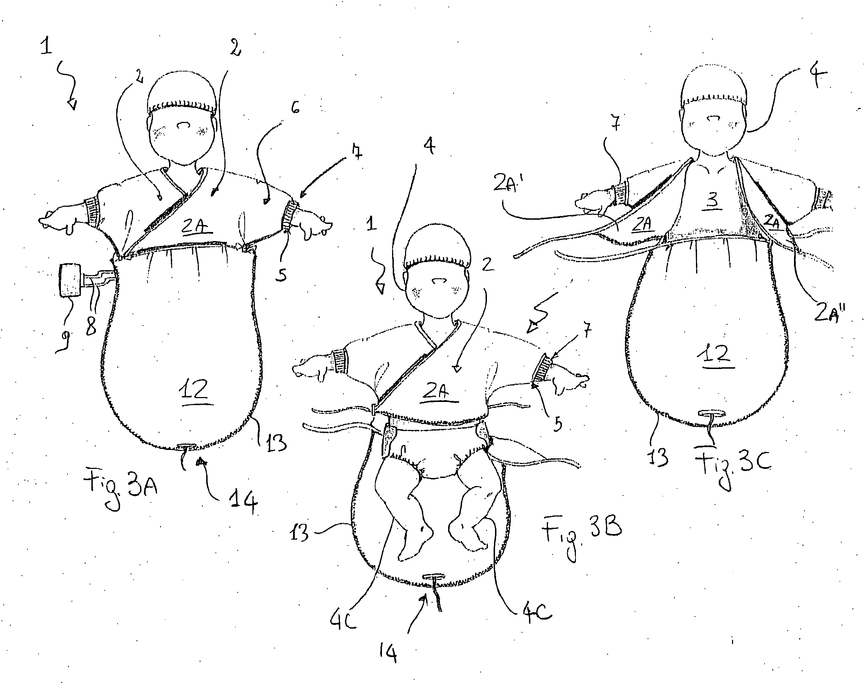

- Figures 3A to 3C show the item of clothing of Figure 1 when worn by a premature baby;
- Figure 4 shows a second embodiment of the item of clothing for detecting vital parameters of a baby, according to the present invention;
- Figure 5 shows a third embodiment of the item of clothing for detecting vital parameters of a baby, according to the present invention;
- Figure 6 shows a fourth embodiment of the item of clothing for detecting vital parameters of a baby, according to the present invention.

### DETAILED DESCRIPTION OF THE INVENTION

Referring to the annexed figures, an item of clothing 1 (hereinafter simply referred to as pyjama) is shown, which is adapted and designed for detection of vital parameters of a baby, preferably a premature baby.

It should be first noted that any person skilled in the art can easily identify the proper size that the item of clothing 1 should have to be uniquely used with a baby, and preferably a premature baby.

This will exclude any item of clothing that can be worn by a baby, but would never be actually used and worn as WHS items of clothing.

Bearing this in mind, the pyjama 1 comprises:
- a first portion 2 adapted to at least partially cover the torso 3 of a baby 4;
- at least one sensor 5 for detecting one or more vital parameters of the baby 4.

The vital parameter/s detected may be ECG recordings, and hence heart rate, respiration rate, oxygen saturation and temperature.

The pyjama 1 is characterized in that it comprises at least one second portion 6 that extends from the first portion 2, such second portion 6 being adapted to at least partially cover one arm 4A of the baby 4.

Preferably, the pyjama 1 comprises a second portion 6 for at least partially covering the left arm and the right arm of the baby 4.

It should be noted that the word cover is intended as wrapping, including or enclosing the torso and/or at least partially the left arm and the right arm of the baby 4.

Particularly, the second portion 6 has a section 7 that is stretchable in at least one direction.

Such stretchable section 7 is designed to remain in a fixed position relative to the arm 4A of the baby 4.

The sensor 5 is advantageously coupled with the stretchable section 7 so that it can be properly positioned relative to the arm of the baby 4 to provide an electrical output signal Sout indicative of the vital parameter being detected.

For this purpose, a connecting cable 8 is provided and it is electrically connected to said sensor 5 to transmit the electrical signal Sout to a data collection unit 9.

The cable 8 is preferably connected to the stretchable section 7.

The data collection unit 9 is operably connected, preferably via Bluetooth wireless communication, to a processing device, such as a personal computer (not shown), having suitable software for performing digital processing on the signal Sout.

It shall be noted that the second portion 6 is preferably formed of one piece with the portion 2, i.e. seamlessly extends from the first portion and has a free end portion 6A.

The second portion 6 preferably extends in the direction of extension of the baby's arm.

Particularly, the second portion 6 consists of a sleeve of the pyjama.

Preferably, the pyjama 1 comprises two sleeves.

Therefore, the pyjama 1 consists of a garment that can cover the chest and arms of the baby and comprises at least one sensor 5 located at the stretchable section 7 which in turn ensures proper placement thereof relative to the arm of the baby 4.

In one preferred embodiment, the stretchable section 7 is located near the free end of the second portion 6, i.e. near the hem of the sleeve.

Otherwise, the stretchable section 7 may be interposed in any position of the second portion 6 starting from the region in correspondence of the shoulder of the baby and ending the free end of said second section 6.

Particularly, the direction of extension of the stretchable section 7 is a direction transverse, preferably perpendicular to the direction of extension of the arm of the baby 4.

It shall be particularly noted that the stretchable section 7 extends in the direction of extension of the arm of the baby 4 to a length L ranging from 0.5 cm to 2.5 cm.

The stretchable section 7 comprises fastening means 11 adapted to be wrapped around the arm of the baby 4.

Such fastening means 11 are selected from the group comprising elastic bands or straps having buttonholes for receiving a button.

The use, for instance, of an elastic band as a fastening means 11, provides the advantage of allowing the stretchable section to be adjusted in their direction of extension, and hence of allowing such stretchable section 7 to be adapted to the particular circumference of the arms of each baby.

Furthermore, this feature allows the conductive fabric (i.e. the sensor 5) to properly adhere, with no pleating, to the skin of the baby, and greatly reduces artifacts caused by electrode-skin impedance.

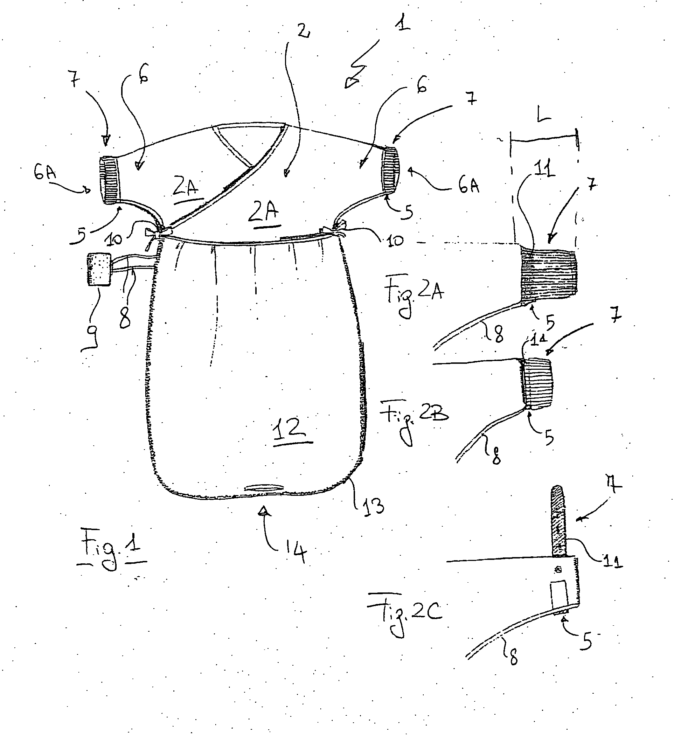

Particularly, also referring to Figures 2A, 2B and 2C, which show different embodiments of the connection between the sensor 5 and the stretchable section 7, it will be noted that:
- in Figure 2A, the pyjama 1 with the sensing fabric is sewn around the elastic cotton, so that it can be adapted to the width of the arm of the baby;
- in Figure 2B, the pyjama 1 is made of sensing fabric in the lower part of the arm, with an upper drawstring;
- in Figure 2C, the pyjama 1 comprises a non-elastic conductive layer as thick as 2 cm, that can be adjusted to the arm of the baby using the outer elastic belt and the buttons that allow sleeve adjustment.

In one aspect of the present embodiment, the at least one sensor 5 is integrated in the stretchable section 7 itself.

Preferably, the at least one sensor 5 extends along an arc of a circle, a half-circle or a circle.

The sensor 5 consists in electrodes woven in the stretchable area 7.

The sensors 5 are made of silver and are connected through two terminals to the data collection unit 9 through a connector, preferably a four-pole connector.

Silver may be contained in the sensor 5 in varying amounts, which will also change the conductivity of the fabric.

Particularly, the smaller the amount of silver, the lower the signal detection sensitivity.

The pyjama 1 may comprise more sensors on the torso 3 or other critical areas of the baby 4, with one sensor being always located on one or both arms of the baby 4.

For example, an accelerometer will be located in a pocket at the first portion, for detecting the movements of the chest of the baby 4.

The first portion 2 comprises a front part 2A and a rear part 2B (not shown), where the front part 2A is adapted to be opened, preferably in the longitudinal direction along the main axis of the baby 4, to uncover the front chest of the baby 4, whereas the rear part 2B is preferably formed of one piece and is adapted to cover the rear chest of the baby.

In one embodiment, the front part 2A comprises two edges 2A' and 2A" which cover the front chest of the baby 4 in juxtaposed or at least partially overlapped relation in a first operating configuration, and can be opened to uncover the chest of the baby in a second operating configuration.

It shall be noted that the two edges 2A' and 2A" are held in the first operating configuration by fastening means 10.

Such fastening means 10 consist of a tape located near the end of the edges 2A' and 2A"; the two tapes are tied together, for instance in a knot.

Alternatively, the fastening means 10 consist of a buttonhole-button assembly.

It will be readily appreciated that the change from the first to the second operating configurations simply requires the knot to be untied or the button to be unbuttoned, for quick access to the front chest of the baby 4 even in emergency conditions, with no interruption of the monitoring activity of the sensor 5, as such sensor is advantageously located on the arm of the baby.

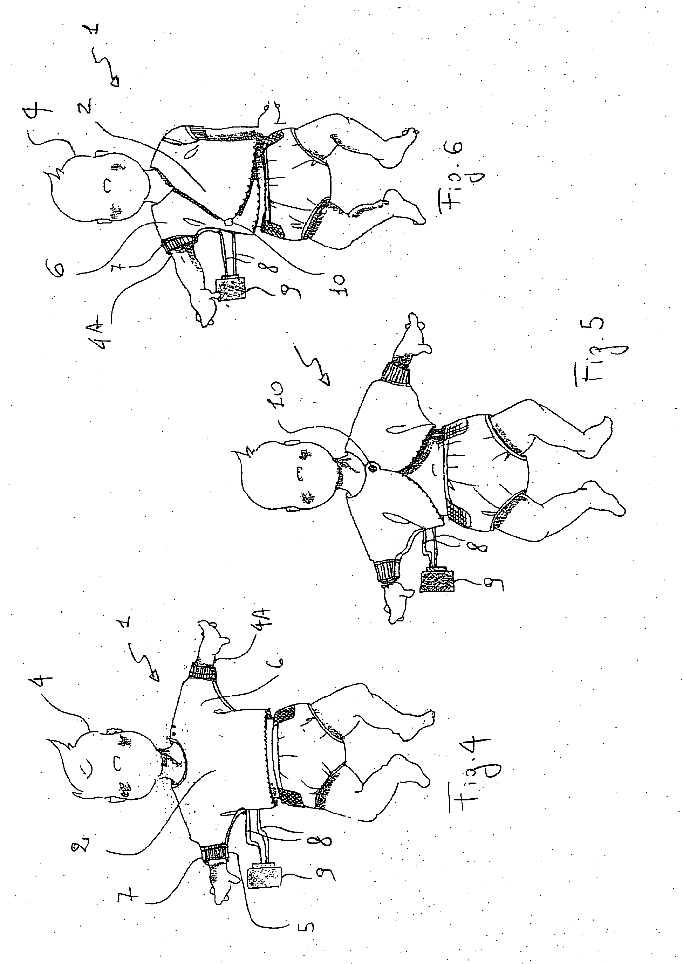

In another embodiment, the front part may be formed of one piece; in this case, the item of clothing 1 will consist of a T-shirt-like garment (see Figure 4).

In a different embodiment, the pyjama 1 may include a third portion 12 extending from the first portion 2.

Such third portion 12 is one embodiment is adapted to create a volume large enough to receive the legs 4C of the baby 4.

Particularly, such third portion 12 preferably forms the extension of the rear part 2B and is folded on itself to form the above mentioned bag.

Preferably, such third portion 12 is formed with the same yarn as the first and second portions 2, 6.

Particularly, seams 13 are preferably provided along the peripheral edge of the bag, obviously excepting the entry side.

These seams are formed so that the seam thread is external to the volume in which the legs 4C of the baby 4 are received.

This advantageously prevents any dangerous abrasion caused by the seam thread rubbing against the baby's skin.

The yarn selected for the pyjama 1 has been made elastic and processed to be soft and harmless for the skin of the baby even in case of rubbing.

Preferably said third portion 12 is made with the same yarn used in the first and second portion 2,6.

It is to be noted that the yarn used for making the pyjama's portions heretofore indicated preferably comprise organic cotton interlock 100% (ICEA certified).

Such organic cotton interlock 100% has advantageously the features of natural softness, anallergicity and it provides the natural transpiration of the skin since it has not been treated with chemical substances besides showing a resistance to multiple washings.

Another important aspect is the position of the connecting cable 8 and the corresponding terminals for interfacing it with the data collection unit 9.

At first, the terminals were directly laid on the sensing section 8 of the pyjama 1, which inevitably created a gap between the baby's skin and the sensor 5; then, a tape was used, with a button at its end, for fastening the terminal.

Later developments allowed the conductive yarn, still highly sensitive to the movements of the arms of the baby, to be extended to the feet of the pyjama, where the terminals were connected (also in view of minimizing bulk in the incubator), where it was disturbed by the movements of the legs.

Finally, the conductive yarn was extended to the waist, where a buttonhole 14 was formed, through which the terminals may be applied with no risk of being moved due to movements of the baby.

The data collection unit 9 consists of electronic hardware adapted for detection of each signal that the sensor/s 5 can acquire.

For this purpose, analog acquisition boards have been provided, which are all contained in the data collection unit 9.

Particularly, the data collection unit 9 allows data acquisition without immediate processing, as it represents an interface between the analog part that detects the signals via the sensors and the digital processing part that extracts the relevant parameters.

This data collection unit 9 comprises a four-pole input, with which the outputs of the temperature sensor and the accelerometer are connected, with the other connector being only left for connection of the channels required for detection of the electrocardiogram ECG.

The data collection unit 9 is battery-powered at 3.3 V, well below the threshold value required for biomedical devices (according to the standard EN60601-1-2 et seq.), by a lithium battery, with 3.7 V rated voltage and 740 mAh.

Those skilled in the art will obviously appreciate that a number of changes and variants may be made to the arrangements as described hereinbefore to meet incidental and specific needs, without departure from the scope of the invention, as defined in the following claims.

## Claims

1. A "baby pyjama" of electronic textile type for a baby (4) being configured for detection of vital parameters of the baby (4), comprising:
- a first portion (2) adapted to at least partially cover the torso (3) of the baby (4);
- at least one sensor (5) for detecting a vital parameter of the baby (4);
- art least one second portion (6) that extends from the first portion (2), said second portion (6) being adapted to at least partially cover one arm (4A) of the baby (4);
- a connecting cable (8) operably connected with said at least one sensor for transmitting an electrical signal (Sout) to a data collection unit (9);
- said at least one second portion (6) having a section (7) that can be stretched in at least one direction, said stretchable section (7) being designed to remain in a fixed position relative to said at least one arm (4A);
- said at least one sensor (5) being integrated in said stretchable section (7) so that it can be properly positioned relative to the arm (4A) of the baby to provide said electrical output signal (Sout) indicative of the vital parameter being detected; **characterized in that:**
- said stretchable section (7) being extended to a range from 0.5cm to 2.5cm in the direction of extension of the arm of the baby and being disposed in correspondence of the shoulder of the baby.

2. A "baby pyjama" of electronic textile type as claimed in claim 1, wherein said first portion (2) comprises a front part (2A) and a rear part (2B), said front part (2A) being adapted to be opened, to uncover the front chest of the baby (4) and said rear part (2B) being adapted to cover the rear chest of the baby (4).

3. A "baby pyjama" of electronic textile type as claimed in claim 1 or 2, wherein said second portion (6) is formed of one piece with said first portion (2), and leaves one end (6A) free, said second portion (6) extending in the direction of extension of the arm of said baby.

4. A "baby pyjama" of electronic textile type as claimed in any one of the preceding claims, wherein said stretchable section (7) is located near the free end (6A) of said second section (6) and its direction of extension is a direction transverse, preferably perpendicular, to the direction of extension of the arm of the baby, said stretchable section comprising first fastening means (11) adapted to be wrapped around said arm.

5. A "baby pyjama" of electronic textile type as claimed in claim 4, wherein said first wrap-around fastening means (11) are selected from the group comprising elastic bands and straps with buttonholes and buttons.

6. A "baby pyjama" of electronic textile type as claimed in claim 2, wherein said front part comprises two edges (2A', 2A") which cover said chest in a first operating configuration, and can be opened in a second configuration, said two edges (2A', 2A") can be in juxtaposed or at least partially overlapped relation.

7. A "baby pyjama" of electronic textile type as claimed in claim 5, wherein said two edges (2A', 2A") are held in said first operating configuration using second fastening means (10).

8. A "baby pyjama" of electronic textile type item of clothing as claimed in claim 1, said at least one sensor (5) extends along an arc of a circle, a half-circle or a circle.

9. A "baby pyjama" of electronic textile type as claimed in claim 1, comprising a third portion (12) extending from said first portion (2), said third portion (12) being adapted to create a bag having a volume large enough to receive the legs (4C) of the baby (4).

## Patentansprüche

1. "Säuglingsschlafanzug" vom Typ eines Elektroniktextils für einen Säugling (4), der zum Detektieren von Vitalparametern des Säuglings (4) gestaltet ist, umfassend:
- einen ersten Abschnitt (2), der geeignet ist, zumindest teilweise den Rumpf (3) des Säuglings (4) zu bedecken;
- mindestens einen Sensor (5) zum Detektieren eines Vitalparameters des Säuglings (4);
- mindestens einen zweiten Abschnitt (6), der sich vom ersten Abschnitt (2) erstreckt, wobei dieser zweite Abschnitt (6) geeignet ist, zumindest teilweise einen Arm (4A) des Säuglings (4) zu bedecken;
- ein Verbindungskabel (8), das betriebsfähig mit dem mindestens einen Sensor verbunden ist, um ein elektrisches Signal (Sout) an eine Datenerfassungseinheit (9) zu übertragen;
- wobei der mindestens eine zweite Abschnitt (6) einen Teil (7) aufweist, der in mindestens einer Richtung gedehnt werden kann, wobei dieser dehnbare Teil (7) gestaltet ist, um in einer festen Lage relativ zu dem mindestens einen Arm (4A) zu bleiben;
- wobei der mindestens eine Sensor (5) derart in den dehnbaren Teil (7) integriert ist, dass er in passender Weise relativ zum Arm (4A) des Säuglings positioniert werden kann, um das elektrische Ausgangssignal (Sout) bereitzustellen, das auf den zu detektierenden Vitalparameter hinweist; **dadurch gekennzeichnet, dass**:
- sich der dehnbare Teil (7) bis zu einem Bereich von 0,5 cm bis 2,5 cm in der Ausdehnungsrichtung des Arms des Säuglings erstreckt und auf Höhe der Schulter des Säuglings angeordnet ist.

2. "Säuglingsschlafanzug" vom Typ eines Elektroniktextils nach Anspruch 1, wobei der erste Abschnitt (2) ein Vorderteil (2A) und ein Hinterteil (2B) umfasst, wobei dieses Vorderteil (2A) geöffnet werden kann, um den vorderen Brustkorb des Säuglings (4) freizulegen, und wobei das Hinterteil (2B) geeignet ist, den hinteren Brustkorb des Säuglings (4) zu bedecken.

3. "Säuglingsschlafanzug" vom Typ eines Elektroniktextils nach Anspruch 1 oder 2, wobei der zweite Abschnitt (6) einstückig mit dem ersten Abschnitt (2) ausgebildet ist und ein Ende (6A) frei lässt, wobei sich der zweite Abschnitt (6) in der Ausdehnungsrichtung des Arms des Säuglings erstreckt.

4. "Säuglingsschlafanzug" vom Typ eines Elektroniktextils nach einem der vorhergehenden Ansprüche, wobei sich der dehnbare Teil (7) nahe dem freien Ende (6A) dieses zweiten Abschnitts (6) befindet und seine Ausdehnungsrichtung eine Richtung ist, die quer, vorzugsweise perpendikulär, zur Ausdehnungsrichtung des Arms des Säuglings ist, wobei der dehnbare Teil erste Befestigungsmittel (11) umfasst, die um diesen Arm gewickelt werden können.

5. "Säuglingsschlafanzug" vom Typ eines Elektroniktextils nach Anspruch 4, wobei die ersten Umwickel-Befestigungsmittel (11) aus der Gruppe ausgewählt sind, die elastische Bänder und Gurte mit Knopflöchern und Knöpfen umfasst.

6. "Säuglingsschlafanzug" vom Typ eines Elektroniktextils nach Anspruch 2, wobei das Vorderteil zwei Ränder (2A', 2A") umfasst, die den Brustkorb in einer ersten Betriebskonfiguration bedecken und die zu einer zweiten Betriebskonfiguration geöffnet werden können, wobei sich diese zwei Ränder (2A', 2A") in einer nebeneinanderliegenden oder zumindest teilweise überlappenden Beziehung befinden können.

7. "Säuglingsschlafanzug" vom Typ eines Elektroniktextils nach Anspruch 5, wobei die zwei Ränder (2A', 2A") unter Verwendung von zweiten Befestigungsmitteln (10) in der ersten Betriebskonfiguration gehalten werden.

8. "Säuglingsschlafanzug" vom Typ eines Elektroniktextil-Kleidungsstücks nach Anspruch 1, wobei sich der mindestens eine Sensor (5) entlang einem Kreisbogen, einem Halbkreis oder einem Kreis erstreckt.

9. "Säuglingsschlafanzug" vom Typ eines Elektroniktextils nach Anspruch 1, der einen dritten Abschnitt (12) umfasst, der sich vom ersten Abschnitt (2) erstreckt, wobei dieser dritte Abschnitt (12) einen Sack bilden kann, dessen Volumen ausreichend groß ist, um die Beine (4C) des Säuglings (4) aufzunehmen.

## Revendications

1. Pyjama pour bébé de type textile électronique pour un bébé (4) qui est configuré pour la détection de paramètres vitaux du bébé (4), comprenant :
- une première portion (2) adaptée pour couvrir au moins partiellement le torse (3) du bébé (4) ;
- au moins un capteur (5) pour détecter un paramètre vital du bébé (4) ;
- au moins une deuxième portion (6) qui s'étend à partir de la première portion (2), ladite deuxième portion (6) étant adapté pour couvrir au moins partiellement un bras (4A) du bébé (4) ;
- un câble de connexion (8) connecté fonctionnellement avec ledit au moins un capteur pour transmettre un signal électrique (Sout) à une unité de récupération de données (9) ;
- ladite au moins une deuxième portion (6) ayant une section (7) qui peut être étirée dans au moins une direction, ladite section étirable (7) étant conçue pour rester dans une position fixe par rapport audit au moins un bras (4A) ;
- ledit au moins un capteur (5) étant intégré dans ladite section étirable (7) de manière qu'il puisse être correctement positionné par rapport au bras (4A) du bébé pour fournir ledit signal électrique de sortie (Sout) indicatif du paramètre vital qui est détecté ; **caractérisé en ce que** :
- ladite section étirable (7) est étendue dans une plage de 0,5 cm à 2,5 cm dans la direction d'extension du bras du bébé et est disposée en correspondance de l'épaule du bébé.

2. Pyjama pour bébé de type textile électronique selon la revendication 1, dans lequel ladite première portion (2) comprend une partie avant (2A) et une partie arrière (2B), ladite partie avant (2A) étant adaptée pour être ouverte, pour découvrir la poitrine du bébé (4) et ladite partie arrière (2B) étant adaptée pour couvrir le dos du bébé (4).

3. Pyjama pour bébé de type textile électronique selon la revendication 1 ou 2, dans lequel ladite deuxième portion (6) est formée d'une seule pièce avec ladite première portion (2), et laisse une extrémité (6A) libre, ladite deuxième portion (6) s'étendant dans la direction d'extension du bras dudit bébé.

4. Pyjama pour bébé de type textile électronique selon l'une quelconque des revendications précédentes, dans lequel ladite section étirable (7) est située près de l'extrémité libre (6A) de ladite deuxième portion (6) et sa direction d'extension est une direction transversale, de préférence perpendiculaire, à la direction d'extension du bras du bébé, ladite section étirable comprenant des premiers moyens de fixation (11) adaptés pour être enveloppés autour dudit bras.

5. Pyjama pour bébé de type textile électronique selon la revendication 4, dans lequel lesdits premiers moyens de fixation enveloppants (11) sont sélectionnés parmi le groupe comprenant des bandes élastiques et des sangles avec des boutonnières et des boutons.

6. Pyjama pour bébé de type textile électronique selon la revendication 2, dans lequel ladite partie avant comprend deux bords (2A', 2A") qui couvrent ladite poitrine dans une première configuration fonctionnelle et peuvent être ouverts dans une deuxième configuration, lesdits deux bords (2A', 2A") peuvent être en relation juxtaposée ou au moins partiellement superposée.

7. Pyjama pour bébé de type textile électronique selon la revendication 5, dans lequel lesdits deux bords (2A', 2A") sont maintenus dans ladite première configuration fonctionnelle en utilisant des deuxièmes moyens de fixation (10).

8. Pyjama pour bébé d'article de vêtement de type textile électronique selon la revendication 1, dans lequel ledit au moins un capteur (5) s'étend suivant un arc de cercle, un demi-cercle ou un cercle.

9. Pyjama pour bébé de type textile électronique selon la revendication 1, comprenant une troisième portion (12) s'étendant à partir de ladite première portion (2), ladite troisième portion (12) étant adaptée pour créer une poche ayant un volume suffisamment grand pour recevoir les jambes (4C) du bébé (4).
